# NEW EUROPEAN PATENT SPECIFICATION

(11) **EP 1 113 771 B2**
(45) Date of publication and mention of the opposition decision: **24.09.2008**
(45) Mention of the grant of the patent: 02.05.2003
(21) Application number: 99941175.4
(22) Date of filing: 13.08.1999
(51) Int. Cl.: A61F 13/15, A61F 13/00

(54) **A METHOD FOR CUTTING AND SEALING AN ABSORBENT MEMBER**
VERFAHREN ZUM SCHNEIDEN UND SCHLIESSEN VON EINEM ABSORBIERENDEN ELEMENT
PROCEDE DE COUPE ET DE SCELLEMENT D'UN ELEMENT ABSORBANT

(30) Priority: 17.08.1998 EP 98115426
(43) Date of publication of application: 11.07.2001
(73) Proprietor: THE PROCTER & GAMBLE COMPANY, Cincinnati, Ohio 45202 (US)
(72) Inventor: HUNDORF, Harald, Hermann, D-61352 Bad Homburg (DE); LINDNER, Torsten, D-61476 Kronberg (DE)
(74) Representative: Kremer, Véronique Marie Joséphine
(86) International application number: PCT/US1999/018596
(87) International publication number: WO 2000/009058

(56) References cited:
- EP-A- 0 030 342
- EP-A- 0 508 485
- EP-A- 0 846 455
- WO-A-93/02861
- US-A- 4 610 678
- US-A- 5 151 092

## Description

This invention relates to a method for wrapping, cutting and sealing an absorbent member, and more particularly to a method for wrapping, cutting and sealing an absorbent member which is suitable for use as an absorbent member in a disposable absorbent article.

### BACKGROUND OF THE INVENTION

Absorbent webs which comprise masses of fibers, i.e., fibrous web, are well known in the art. Such webs can imbibe liquids, such as discharged body fluids, both by an absorption mechanism wherein fluid is taken up by the fiber material itself and by a wicking mechanism wherein fluid is acquired by, distributed through and stored in the capillary interstices between fibers. One means for improving the absorbency characteristics of such fibrous web structures is to incorporate therein superabsorbent material, such as polymeric gelling material (also referred to as hydrogel-forming material superabsorbent polymers, etc.) which imbibe fluid. The superabsorbent material serves to retain fluid such as discharge body liquids. An absorbent structure of this type wherein hydrogel-forming materials in particulate form are incorporated into fibrous webs is disclosed in Weisman and Goldman, U.S. Patent 4,610,678, issued September 9, 1986.

The improvement in absorbency provided by incorporation of absorbent gelling materials has permitted the realization of absorbent articles, such as disposable diapers, which employ relatively thin absorbent members and which are, therefore, relatively thin products.

Notwithstanding the existence of absorbent members as described above, there remains a need to provide absorbent members which reduce and preferably eliminate the phenomena referred to as gel-on-skin. Gel-on-skin is the situation where absorbent gelling materials escape from the absorbent member and travel through the bodyside liner or topsheet of the absorbent article where they come into contact the wearer's skin.

In prior art continuous lay down operations, fibers and superabsorbent materials are mixed together in a continuous web. The continuous web is then cut into individual absorbent members or cores. The individual absorbent members are then placed between a liquid pervious topsheet and a liquid impervious backsheet to form an absorbent article. Unfortunately, this configuration provided an unsatisfactory product as absorbent gelling material easily penetrated through the topsheet creating unacceptable amounts of gel-on-skin.

One solution to the above continuous lay down operation, was to place another web, such as a tissue or nonwoven web on top of the continuous web and then cut both the tissue and continuous web into individual members comprising the core and the tissue. The individual members were then placed in the product with the tissue positioned between the topsheet and the absorbent member substantially preventing absorbent gelling material from escaping from the uppermost surface of the absorbent member and thus reducing the amount of gel-on-skin.

Unfortunately, when for example, the tissue and the continuous web are cut into individual members, the ends of the absorbent member are left open, i.e., the ends of the absorbent member are not covered by the tissue, allowing absorbent gelling material to escape through the ends of the absorbent member.

EP 0 846 455 A1 discloses a process for manufacturing individual layered structures comprising a step of applying a thermoplastic material to the continuous web like layered struture over at least part of the designated cutting regions. EP 0 846 455 A1 does not, however, disclose the use of a wrap web material.

It is an object of this invention to provide a method of cutting and sealing an absorbent member via a continuous lay down operation which circumvents the problems of gel-on-skin.

### BRIEF SUMMARY OF THE INVENTION

The invention is a method for wrapping, cutting and sealing an absorbent member. The absorbent member comprises a core web and a wrap web. The core web comprises a fibrous material and superabsorbent material and has a first side edge and a second side edge, the wrap web has an inner surface and an outer surface. The method of the present invention comprises the steps of applying a superabsorbent material movement obstruction agent to the wrap web, combining the wrap web with the core web such that the inner surface of the wrap web is facing towards the core web to form an absorbent member web, folding the wrap web around the first side edge and/or the second side edge of the core web, cutting the absorbent member web into discrete absorbent members.

The superabsorbent material movement obstruction agent is a material selected from the group of polymeric solutions or emulsions.

The individual absorbent members preferably form an absorbent member in a disposable absorbent article and are positioned between a liquid pervious topsheet and a liquid impervious backsheet.

### BRIEF DESCRIPTION OF THE DRAWINGS

While the specification concludes with claims pointing out and distinctly claiming the present invention, it is believed the same will be better understood by the following drawings taken in conjunction with the accompanying specification wherein like components are given the same reference number.

Figure 1 is a plan view of an absorbent article comprising an absorbent member manufactured according to the process of the present invention.

Figure 2 is a schematic, perspective view of a first embodiment of the method of the present invention.

Figure 3 is a schematic, perspective view of a second embodiment of the method of the present invention.

### DETAILED DESCRIPTION OF THE INVENTION

The method of the present invention is particularly suitable for manufacturing absorbent members for use in disposable absorbent articles. As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A "unitary" absorbent article refers to absorbent articles which are formed of separate parts united together to form a coordinated entity so that they do not require separate manipulative parts like a separate holder and liner. As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

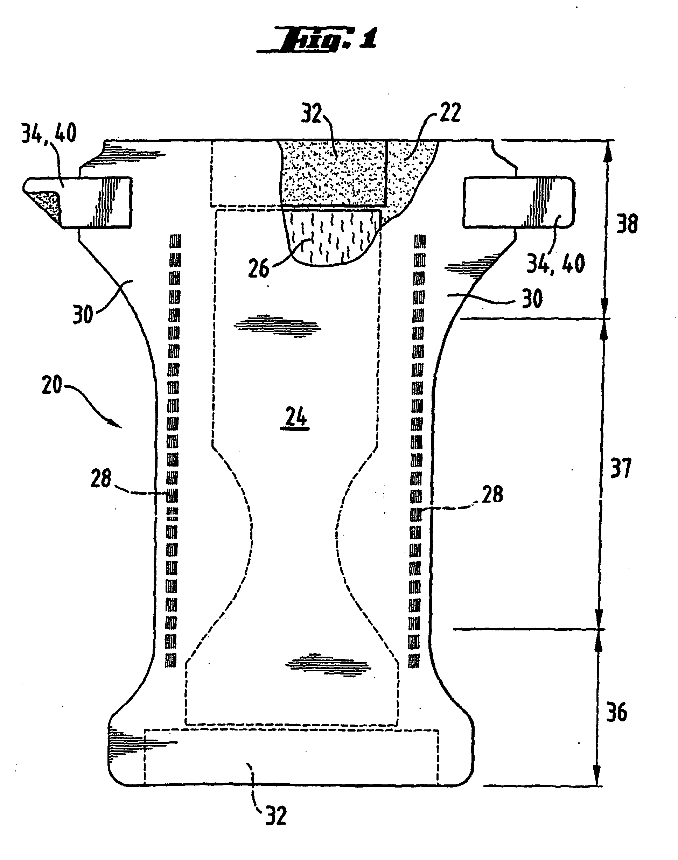

A preferred embodiment of a unitary absorbent article comprising an absorbent member manufactured by the method of the present invention is the unitary disposable absorbent article, diaper 20, shown in Figure 1. As used herein, the term "diaper" refers to an absorbent article generally worn by infants and adult incontinent persons and is worn about the lower torso of the wearer. It should be understood, however, that the present invention is also applicable to other absorbent articles such as incontinence briefs, incontinence undergarments, absorbent inserts, diapers holders and liners, feminine hygiene garments, and the like.

With reference to Figure 1, an absorbent article, such as a diaper 20, generally comprises a liquid pervious topsheet 22, a liquid impervious backsheet 24 joined with the topsheet 22; and an absorbent member 26 intermediate the topsheet 22 and the backsheet 24. The diaper 20 preferably further comprises a front waist region 36, a rear waist region 38, a crotch region 37 positioned between the front waist region 36 and the rear waist region 38, elasticized leg cuffs 28, ear flaps 30, an elastic waist feature 32 and a fastening system 34 comprising at least one tape tab 40. An example of a suitable absorbent article to which the absorbent member of the present invention may be inserted is more fully and completely described in U.S. Patent No. 5 151 092 issued to Buell et al. on September 29, 1992.

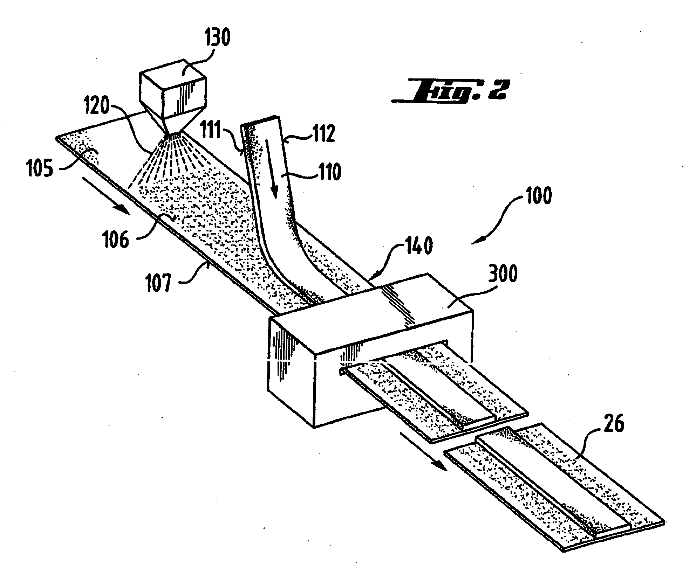

The absorbent member 26 of the present invention may be produced on the apparatus 100, as shown in Figure 2. In a preferred embodiment, the apparatus 100 is integrated into a disposable absorbent article manufacturing line such that the absorbent member 26 of the present invention may be manufactured "on-line". (As used herein, the term "integrated" means interconnected process modules that operate concurrently to produce finished products from source materials. The term "on-line" is used to refer to the process of manufacturing the absorbent members of the present invention on an apparatus that is integrated with the manufacturing line that produces the disposable absorbent articles to which the tape tabs will be joined.)

The absorbent member 26 of the present invention comprises a core member and a wrap member being cut from a core web 110 and a wrap web 105 respectively during the manufacture of the absorbent member 26 of the present invention.

Examining apparatus 100 in greater detail, a wrap web 105 having an inner surface 106 and an outer surface 107 is provided. A superabsorbent material movement obstruction agent 120 is applied to the wrap web 105 by a superabsorbent material movement obstruction agent dispensing device 130. Preferably, the superabsorbent material movement obstruction agent 120 is applied to the inner surface 106 of the wrap web 105. A core web 110 having a first side edge 111 and a second side edge 112 is provided and combined with the wrap web 105 such that the inner surface 106 is facing towards the core web 110, thereby forming an absorbent member web 140. The absorbent member web 140 is fed into a cutting device 300 which cuts the absorbent member web into discrete absorbent members 26.

The core web 110 comprises fibrous material and superabsorbent material. The fibrous material may comprise cellulose fibers, in the form of fluff; modified cellulose fibers such as stiffened cellulose fibers; synthetic fibers such as those made of cellulose acetate, polyvinyl fluoride, polyvinylidene chloride, acrylics, polyvinyl acetate, non-soluble polyvinyl alcohol, polyethylene, polypropylene, polyamides (such as nylon), polyesters, bi-component fibers, tricomponent fibers, mixtures thereof and the like. Preferred synthetic fibers have a denier of from about 3 denier per filament to about 25 denier per filament, more preferably from about 5 denier per filament to about 15 denier per filament. Also preferably, the fiber surfaces are hydrophilic or are treated to be hydrophilic.

Suitable superabsorbent materials include but are not limited to discrete particles of absorbent gelling material and superabsorbent fibrous material such as acrylate grafted fibers and superabsorbent modified fibers. The superabsorbent material can be in any form which can be incorporated into a flexible web or sheet to form the web 110. The superabsorbent material, upon contact with fluids such as water or body fluids, absorb such fluids. The superabsorbent material is typically in the form of discrete particles of absorbent gelling material.

The superabsorbent material movement obstruction agent 120 is a material selected from the group of polymeric solutions or emulsions, both natural (e.g. natural rubber latex) and synthetic, in which the liquid is water or any other suitable liquid or mixture of liquids. Waterborne emulsions are preferred and more preferred are waterborne emulsions of acrylic or vinylic adhesive polymers.

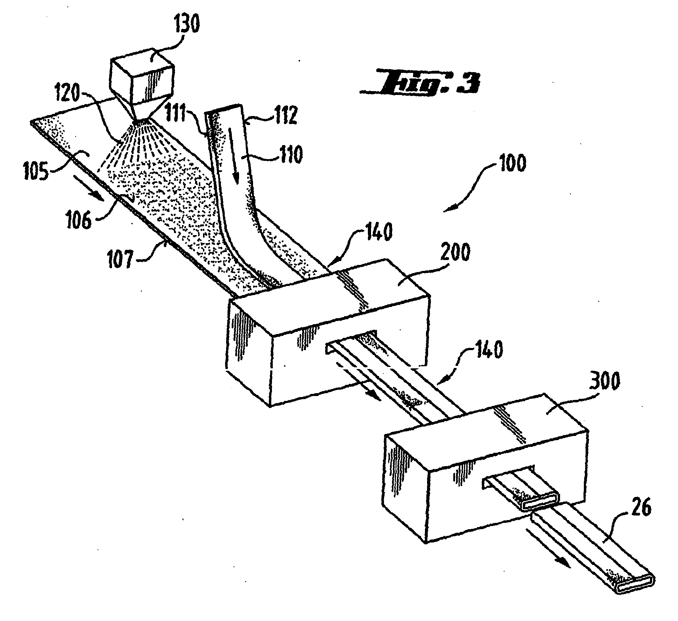

Figure 3 shows an alternative embodiment of the method of the present invention further comprising a step of folding the wrap web 105 around the first side edge 111 and/or the second side edge 112 of the core web 110 by the folding device 200. Preferably, the first side edge of the wrap web is joined to the second side edge of the wrap web after the folding such that the wrap web transversely envelopes the core web.

The superabsorbent material movement obstruction agent is preferably not applied to the entire wrap web, but only in discrete, spaced apart zones. While the superabsorbent material movement obstruction agent does provide the benefit of obstructing the movement of the superabsorbent material through the cut end of the absorbent members, it may have some negative effects if applied to the entire wrap web. For example, the agent may increase the stiffness of the absorbent member such that it becomes uncomfortable for the wearer if applied to the entire wrap web. The agent may inhibit some of the absorbent properties of the absorbent member and thus would negatively impact the absorbent article which employed an absorbent member having the agent applied to the entire wrap web. Thus, in order to achieve the desired effect of obstructing the movement of the superabsorbent material through the cut end of the absorbent members without negatively impacting the performance, comfort or other properties and characteristics of the absorbent members and an absorbent article which employs such a member, the superabsorbent material movement obstruction agent is applied to the wrap web in only discrete, spaced apart zones. Preferably, the zones extend over the entire width of the wrap web.

In another embodiment of the method of the present invention, the absorbent member web comprising a wrap web a core wrap is combined with a second wrap web having an inner surface and an outer surface such that the core web is positioned intermediate the wrap web and the second web, the inner surface of the second wrap web facing towards the core web. The wrap web and/or the second wrap web preferably are folded around the first side edge and/or the second side edge of the absorbent web such that the side edges of the wrap web and the second wrap web overlap. More preferably, the side edges of the first wrap web and the second wrap web are joined to each other to transversely envelope the core web.

Preferably, superabsorbent material movement obstruction agent is additionally applied to the second web, more preferably to the inner surface of the second wrap web.

## Claims

1. A method for cutting and sealing an absorbent member (26), said absorbent member (26) comprising a core web (110) comprising a fibrous material and a superabsorbent material and having a first side edge (111) and a second side edge (112), a wrap web (105) having an outer surface 10 and an inner surface (106), said method being **characterized by** the steps of:
- applying a superabsorbent material movement obstruction agent (120) to said wrap web (105), wherein said superabsorbent material movement obstruction agent (120) is a material selected from the group of polymeric solutions or emulsions,
- combining said wrap web (106) with said core web (110) such that said Inner surface (106) is facing towards said core web (116) to form an absorbent member web (140),
and said method further comprising a step of
- cutting said absorbent member web (140) Into discrete absorbent member (28).

2. The method of Claim 1 wherein said superabsorbent material movement obstruction agent (120) is applied to the inner surface (106) of said wrap web (105).

3. The method of Claim 1 further comprising a step of folding said wrap web (105) around said first side edge (111) and/or said second side edge (112).

4. The method of Claim 3 wherein said wrap web (105) has a first side edge and a second side edge and the method further comprises the step of transversely enveloping said core web (110) by joining said first side edge of said wrap web (105) to said second side edge of said wrap web (105).

5. The method of Claim 1 wherein said wrap web (105) has a first side edge and a second side edge and said method further comprises the steps of providing a second wrap web having an Inner surface and an outer surface, transversely enveloping said core web (110) by joining said first side edge of said wrap web (105) and said second side edge of said wrap web (105) to said second wrap web such that said Inner surface of said second wrap web Is facing towards said core web (110).

6. The method of Claim 5 wherein said method further comprises the step of applying a superabsorbent material movement obstruction agent (120) to said second wrap web.

7. The method of Claim 6 wherein said superabsorbent material movement obstruction agent (120) is applied to said inner surface of said second wrap web.

8. The method of any preceding claim wherein said polymeric solutions or emulsions are either natural or synthetic.

## Patentansprüche

1. Verfahren zum Schneiden und Abdichten eines absorbierenden Elements (26), wobei das absorbierende Element (26) eine Kernbahn (110) umfaßt, die ein faseriges Material und ein superabsorbierendes Material aufweist und einen ersten Seitenrand (111) und einen zweiten Seitenrand (112), eine Hüllbahn (105) mit einer äußeren Oberfläche (107) und einer inneren Oberfläche (106) hat, wobei das Verfahren **gekennzeichnet ist durch** die Schritte:
- Aufbringen eines Bewegungsblockierungsmittels (120) für ein superabsorbierendes Material auf die Hüllbahn (105), in welchem das Bewegungsblockiermittel (120) für superabsorbierendes Material ein Material ist, ausgewählt aus der Gruppe von polymeren Lösungen oder Emulsionen,
- Kombinieren der Hüllbahn (105) mit der Kernbahn (110), derart, daß die innere Oberfläche (106) der Kernbahn (116) zugewandt ist, um eine absorbierende Elementbahn (140) zu bilden,
und das Verfahren ferner einen Schritt aufweist
- Schneiden der absorbierenden Elementbahn (140) in diskrete absorbierende Elemente (26).

2. Verfahren nach Anspruch 1, in welchem das Bewegungsblockiermittel (120) für superabsorbierendes Material auf der inneren Oberfläche (106) der Hüllbahn (105) aufgebracht wird.

3. Verfahren nach Anspruch 1, ferner mit einem Schritt des Faltens der Hüllbahn (105) um den ersten Seitenrand (111) und/oder dem zweiten Seitenrand (112) herum.

4. Verfahren nach Anspruch 3, in welchem die Hüllbahn (105) einen ersten Seitenrand und einen zweiten Seitenrand hat und das Verfahren ferner umfaßt den Schritt des quer Einschlagens der Kernbahn (110) durch Verbinden des ersten Seitenrandes der Hüllbahn (105) mit dem zweiten Seitenrand der Hüllbahn (105).

5. Verfahren nach Anspruch 1, in welchem die Hüllbahn (105) einen ersten Seitenrand und einen zweiten Seitenrand hat und das Verfahren ferner die Schritte umfaßt des Bereitstellen einer zweiten Hüllbahn mit einer inneren Oberfläche und einer äußeren Oberfläche, ein quer Einschlagen der Kernbahn (110) durch Verbinden des ersten Seitenrandes der Hüllbahn (105) und des zweiten Seitenrandes der Hüllbahn (105) mit der zweiten Hüllbahn, derart, daß die innere Oberfläche der zweiten Hüllbahn der Kernbahn (110) zugewandt ist.

6. Verfahren nach Anspruch 5, in welchem das Verfahren ferner den Schritt umfaßt des Aufbringens eines Bewegungsblockiermittels (120) für ein superabsorbierendes Material auf die zweite Hüllbahn.

7. Verfahren nach Anspruch 6, in welchem das Bewegungsblockiermittel (120) für superabsorbierendes Material auf der inneren Oberfläche der zweiten Hüllbahn aufgebracht wird.

8. Verfahren nach einem vorhergehenden Anspruch, in welchem die polymeren Lösungen oder Emulsionen entweder natürlich oder synthetisch sind.

## Revendications

1. Procédé pour découper et sceller un élément absorbant (26), ledit élément absorbant (26), comportant une nappe d'âme (110) comprenant un matériau fibreux et un matériau superabsorbant, et présentant un premier bord latéral (111) et un deuxième bord latéral (112), une nappe d'enveloppe (105) présentant une surface extérieure (107) et une surface intérieure (106), ledit procédé étant **caractérisé par** les étapes consistant à :
- appliquer un agent d'obstruction de mouvement (120) du matériau superabsorbant à ladite nappe d'enveloppe (105), lequel agent d'obstruction de mouvement (120) du matériau superabsorbant est un matériau choisi dans le groupe des solutions ou des émulsions polymères,
- combiner ladite nappe d'enveloppe (105) avec ladite nappe d'âme (110) de façon que ladite surface intérieure (106) soit orientée en direction de ladite nappe d'âme (116) afin de former une nappe d'élément absorbant (140),
et ledit procédé comprenant, en outre, l'étape consistant à :
- découper ladite nappe d'élément absorbant (140) en éléments absorbants distincts (26).

2. Procédé selon la revendication 1, dans lequel ledit agent d'obstruction de mouvement (120) du matériau superabsorbant est appliqué à la surface intérieure (106) de ladite nappe d'enveloppe (105).

3. Procédé selon la revendication 1, comprenant, en outre, une étape consistant à plier ladite nappe d'enveloppe (105) autour dudit premier bord latéral (111) et/ou dudit deuxième bord latéral (112).

4. Procédé selon la revendication 3, dans lequel ladite nappe d'enveloppe (105) présente un premier bord latéral et un deuxième bord latéral, et le procédé comprend, en outre, l'étape consistant à envelopper transversalement ladite nappe d'âme (110) en réunissant ledit premier bord latéral de ladite nappe d'enveloppe (105) audit deuxième bord latéral de ladite nappe d'enveloppe (105).

5. Procédé selon la revendication 1, dans lequel ladite nappe d'enveloppe (105) présente un premier bord latéral et un deuxième bord latéral, et ledit procédé comprend, en outre, les étapes consistant à fournir une deuxième nappe d'enveloppe présentant une surface intérieure et une surface extérieure, à envelopper transversalement ladite nappe d'enveloppe (110) en réunissant ledit premier bord latéral de ladite nappe d'enveloppe (105) et ledit deuxième bord latéral de ladite nappe d'enveloppe (105) à ladite deuxième nappe d'enveloppe afin que ladite surface intérieure de ladite deuxième nappe d'enveloppe soit orientée en direction de ladite nappe d'âme (110).

6. Procédé selon la revendication 5, dans lequel ledit procédé comprend, en outre, l'étape consistant à appliquer un agent d'obstruction de mouvement (120) du matériau superabsorbant à ladite deuxième nappe d'enveloppe.

7. Procédé selon la revendication 6, dans lequel ledit agent d'obstruction de mouvement (120) du matériau superabsorbant est appliqué à ladite surface intérieure de ladite deuxième nappe d'enveloppe.

8. Procédé selon l'une quelconque des revendications précédentes, dans lequel lesdites solutions ou émulsions polymères sont naturelles ou synthétiques.
